(19) Europäisches Patentamt
European Patent Office
Office européen des brevets

(11) Publication number: **0 677 292 A1**

# (12) EUROPEAN PATENT APPLICATION

(21) Application number: **95301797.7**

(51) Int. Cl.$^6$: **A61K 31/19**

(22) Date of filing: **17.03.95**

(30) Priority: **17.03.94 GB 9405292**
**18.05.94 TR 393/94**

(43) Date of publication of application:
**18.10.95 Bulletin 95/42**

(84) Designated Contracting States:
**AT BE CH DE DK ES FR GB GR IE IT LI LU MC NL PT SE**

(71) Applicant: **RADOPATH LIMITED**
**17 Bond Street,**
**St.Helier**
**Jersey (GB)**

(72) Inventor: **Ayuko, Washington Odur**
**25 Sundridge Road,**
**Kingstanding**
**Birmingham B44 4NY (GB)**

(74) Representative: **Lawrence, Malcolm Graham**
**Hepworth, Lawrence, Bryer & Bizley**
**Merlin House**
**Falconry Court**
**Bakers Lane**
**Epping Essex CM16 5DQ (GB)**

## (54) Anti-viral and anti-cancer agents.

(57) 2-chloro-5-nitrobenzoic acid and various other chloro and nitro substitute benzoic acid derivatives are disclosed for administration in tablet or injectable solution form for the prophylaxis and therapy of neoplasm or viral infection. The active compounds have surprising efficacy in various treatment regimes and have particular application to HIV-infection and demonstrate various beneficial effects, including regression of lesions in HIV-related Kaposi's sarcoma.

*FIG.1*

AZT AND ddCyd INTERNAL CONTROLS

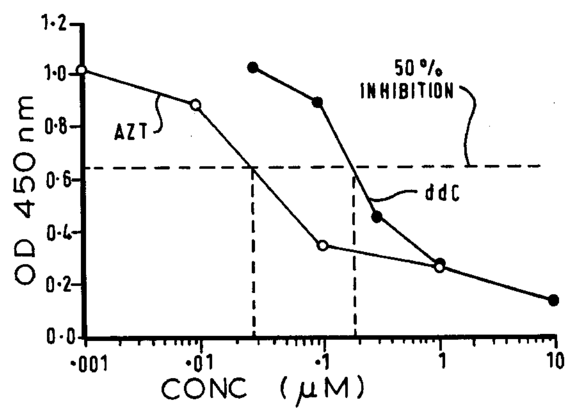


EP 0 677 292 A1

Jouve, 18, rue Saint-Denis, 75001 PARIS

The present invention relates to arylating agents, in particular phenylating agents, which are suitable as therapeutic compounds, especially in the treatment of cancer and disease caused by viral infections (eg retrovirus infections such as HIV. As shown by studies reported herein, the compounds of the invention show marked anti-tumour activity and in AIDS and pre-AIDS therapy show the capacity to regress Kaposi's sarcoma, improve CD4 count in humans, increase the level of various blood parameters in murine patient models and to achieve weight gain and general patient condition by action against HIV and/or its effects (eg opportunistic disease).

According to the invention, there is provided a compound of the following general formula for use in the treatment of prophylaxis or therapy of neoplasm or viral infection such as HIV-infection:-

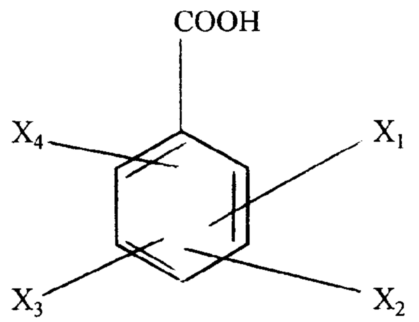

I

wherein $X_1$ and $X_2$ are, each independently, hydrogen or chloro and wherein $X_3$ and $X_4$ are, each independently, hydrogen or nitro, provided that at least two of $X_1$, $X_2$, $X_3$ and $X_4$ are other than hydrogen, or a salt or ester thereof (eg a salt wherein -COOH is substituted by -COOR in which R is optionally substituted amino).

Conveniently, $X_1$ is chloro, $X_2$ is chloro or hydrogen, $X_3$ is nitro and $X_4$ is nitro or hydrogen. The following are preferred compounds:-

2, 4-dichloro-3, 5-dinitrobenzoic acid
4-chloro-3, 5-dinitrobenzoic acid
2-chloro-3, 5-dinitrobenzoic acid
2-chloro-5-nitrobenzoic acid
2, 5-dichlorobenzoic acid
2, 4-dinitrobenzoic acid
3, 5-dinitrobenzoic acid
2, 5-dichloro-4-nitrobenzoic acid
2, 4-dichloro-5-nitrobenzoic acid
2, 6-dichloro-4-nitrobenzoic acid
3, 5-dichloro-4-nitrobenzoic acid
4-chloro-3-nitrobenzoic acid
2-chloro-4-nitrobenzoic acid
3, 4-dichlorobenzoic acid

Since treatment in accordance with the invention is by what is believed to be an arylating mechanism, use is typically at relatively high concentrations and, consequently, doses. Generally, the recommended dosage is 1mg/kg body weight to 30mg/kg body weight per day for 5 days weekly.

The compounds of the invention may be prepared by known process techniques for preparing benzene substituted compounds. Such techniques are described in various standard texts, for example, "Organic Syntheses" 1963 Collective Volume 4, pages 364 to 366, by Harry P Schultz and published by John Wiley and Sons Inc. Post-preparation sterilization may be required, at least following formulation of the compounds into pharmaceutical composition form.

The compounds of the invention may be formulated for use as pharmaceutical compositions (eg for iv, ip, oral or sc administration) comprising at least one active compound and a diluent or carrier. Thus, the invention includes a pharmaceutical composition, which composition comprises a compound according to the invention and a pharmaceutically-acceptable diluent or carrier (eg aqueous).

Such a composition may be in bulk form or, more preferably, unit dosage form. Thus, for example, the composition may be formulated as a tablet, capsule, powder, solution or suspension. Soft gel capsules may be especially convenient. The composition may be a liposomal formulation or administered in a slow sustained release delivery system.

Compositions in accordance with the invention may be prepared using the active compounds defined herein in accordance with conventional pharmaceutical practice. The diluents, excipients or carriers which may be used are well known in the formulation art and the form chosen for any particular regimen will depend on the given context and the physician's choice.

Thus, for example, as illustrated below the compounds of the invention may be administered in solution in sterile deionised water. Also, if necessary, solution may be facilitated using dimethyl sulphoxide (DMSO) or alternatively an alcohol, a glycol or a vegetable oil. The compounds are most favourably administered in corn oil or as a solution in DMSO/sterile water. In the case of the use according to the invention of sodium salts of the active compounds, the active ingredient is preferably administered as an aqueous solution.

In using a compound of the invention, dosage guidance can be taken from animal studies such as that described below. In such studies doses of from about 50mg/kg typically up to about 1000mg/kg (eg up to about 400mg/kg, conveniently about 200mg/kg or less). Thus it is to be expected that a typical dosage for humans will be from about 5mg/kg upwards (eg up to about 20mg/kg). The concentration and dose are to be sufficient to bring an arylating mechanism into play. For intravenous administration the active compound will be dissolved or dispersed in an aqueous or other injectable liquid bearing in mind the aforementioned requirements of dosage. Typically, concentration will be 250mg/ml or less. Patient tolerance was found to be of a higher order for concentrations below that threshold although concentrations of more than 250mg/ml may be used. Typically, a preferred concentration is less than 100mg/ml, with concentrations below 50mg/ml (eg 40mg/ml) being preferred. For oral administration, enteric coated tablets will often be preferred. Maximum strength will be less than 500mg and preferably 400mg or less. Thus, for example, a range of strengths for oral administration (whether in tablet or other solid form) will be 100mg, 200mg or 400mg. Patients who are subject to oral administration will generally fast for 8 hours prior to first dose with the fast continuing for 4 hours after first dose. Free access to fluid will generally be allowed during this fasting period. Intravenous treatment will generally take place by administration very slowly over a period, typically a period of approximately 20 minutes or more. Administration will typically be by catheter, for example a catheter left in situ after flashing with 2ml of saline. Of course, the catheter will in practice then be used for taking blood samples for pharmacokinetic measurements and removed prior to the patient's departure from the clinic.

As shown by the results reported in Table 6 below, 2-chloro-5-nitrobenzoic acid shows considerable anti-tumour activity in vivo. This could not be supported in vitro and it appears some compounds according to the invention require activation in the patient's liver. This and some other compounds may also be immunomodulators.

The following animal study illustrates the remarkable activity of compounds of the invention.

## ANIMAL STUDIES

The in vivo anti-tumour responses of various compounds according to the invention were assessed against the ascitic tumours, MAC15A murine colon adenocarcinoma and various solid tumour models. The MAC15A ascites tumour cells were transplanted into male NMR1 mice by ip innoculation at a cell density of $1 \times 10^5$ cells in 200μl buffer (Table 1). The solid tumour models included the MAC13 and MAC16 murine colon adenocarcinomas, the B16 F1 murine melanoma and the M5076 reticulum cell sarcoma.

Treatment commenced 3 days after ip transplant or, in the case of solid tumours such as MAC13 and MAC16, treatment commenced when average tumour volumes reached $40mm^3$.

The animals were located in both cases into groups of 5 to 8 animals.

The animals were sacrificed after 12 days or when tumours ulcerated, tumour volume exceeded $1000mm^3$ or loss of body weight exceeded 50%.

Except where otherwise stated, the compounds used were dissolved in DMSO and diluted in sterile distilled water, at appropriate concentrations before administration in a solvent volume of 200μl. Anti-tumour responses were obtained by comparing the median survival times or tumour growth inhibition against solvent controls. The results obtained are as shown in Tables 1 to 6 below.

## Preparation

Preparation of dosage solutions is exemplified as follows:-

| | | |
|---|---|---|
| Subjects: | No : | 10 animals |
| | Weight: | 22g |
| Dosage: | 50mg/kg weight per animal per day thus 1.1mg per mouse per day | |
| Total Mass Dosage: | 55mg active ingredient (referred to 5 day treatment regime) | |
| Total Formulation: | 10ml solvent plus 55mg for division into 50 doses of 1.1mg dissolves in 200μl solvent | |

T/C%

T/C% is determined as follows:-

| Animal Survival | Test | Control |
|---|---|---|
| | T days | C days |

$$T/C\% = \frac{T}{C} X 100$$

Example

| Animal Survival | Test | Control |
|---|---|---|
| | 443 days | 100 days |

$$T/C\% = \frac{443}{100} X 100 = 443$$

A figure of 158 or above indicates performance justifying clinical trial.

Conclusions

The effect of a group of compounds on the growth rate of a number of experimental tumours has been evaluated in vivo and the following findings were noted:-

1. Structure-activity relationships against the MAC15A murine colon adenocarcinoma, in the female NMRI mice showed maximal activity on a split-dose schedule and when the halogen was maximally activated for nucleophilic attack.

2. Against the M5076 reticulum cell sarcoma, 2, 4-dichloro-3, 5-dinitrobenzoic acid showed activity on a split-dose schedule down to 25mg/kg body weight by both ip and sc routes. Both the amide and the methyl ester showed 10-fold increase in toxicity and were without anti-tumour activity. The acid also effectively inhibited growth of B16 murine melanoma and the MAC16 murine colon adenocarcinoma.

It is concluded that this group of compounds show a wide spectrum of activity against murine models.

TABLE 1

Anti-tumour activity against MAC15A (murine adenocarcinoma colon). 5 animals per group. Dose 100mg $kg^{-1}$ ip per day.

| Code | Compound | Schedule (days) | T/C%[a] |
|---|---|---|---|
| C4 | 4-chloro-3, 5-dinitrobenzoic acid | 1, 2, 3, 4, 5 | 271 |
| C1 | 2, 4-dichloro-3, 5-dinotrobenzoic acid | 1, 2 | 243 |
| C12 | 2, 5-dichlorobenzoic acid | 1, 2, 3, 4, 5 | 171 |
| C28 | 2, 4-dinitrobenzoic acid | 1, 2, 3, 4, 5 | 100 |
| C29 | 3, 5-dinitrobenzoic acid | 1, 2, 3, 4, 5 | 100 |

a = median, T-test group, C-solvent control

TABLE 2

Anti-tumour activity against M5076-reticulum cell sarcoma 16 days after im transplant. 7 animals per group. Drugs dissolved in corn oil. Dosage is per day.

| Compound | Dose (mg/kg) | Route | Schedule (days) | % Tumour Weight Inhibition |
|---|---|---|---|---|
| 2, 4 BA | 75[a] | ip | 1, 4, 6, 9 | 79, 88[b] |
| | 50 | ip | 1, 4, 6, 9 | 57 |
| | 25 | ip | 1, 2, 4, 6, 9 | 75 |
| | 75 | sc | 1, 4, 5, 7, 9 | 66 |
| | 50 | sc | 1, 2, 4, 5, 6, 7, 9 | 76 |
| | 25 | sc | 1, 2, 4, 5, 6, 7, 9 | 63 |
| 2, 4 BM | 1.0[a] | ip | 1, 2, 3, 4, 5, 6, 7, 8, 9 | 41 |
| | 0.5 | ip | 1, 2, 3, 4, 5, 6, 7, 8, 9 | 39 |
| | 0.25 | ip | 1, 2, 3, 4, 5, 6, 7, 8, 9 | 42 |

a = Maximum tolerated dose

b = Two independent experiments; 4 animals had no tumour in the second experiment

2, 4 BA = 2, 4-dichloro-3, 5-dinitrobenzoic acid (C1)

2,4 BM = 2, 4-dichloro-3, 5-dinitrobenzoic acid methyl ester (C3)

% Tumour Weight Inhibition:-

| Treated | Control | |
|---|---|---|
| Agm | Bgm | Tumour weight |

$$\% \text{ inhibition} = \frac{B - A}{B} \times 100$$

TABLE 3

Anti-tumour activity against B16F1-murine melanoma 12 days after sc transplant. 6 animals per group. Drugs dissolves in corn oil. Dosage is per day.

| Compound | Dose (mg/kg) | Route | Schedule (days) | % Tumour Weight Inhibition |
|---|---|---|---|---|
| 2, 4 BA | 75[a] | ip | 1, 5 | 71, 81[b] |
| | 50 | ip | 1, 5 | 45, 56[b] |
| | 25 | ip | 1, 5 | 13 |
| | 75 | sc | 1, 3, 5 | 30 |
| | 50 | sc | 1, 3, 5 | 9 |
| | 25 | sc | 1, 3, 5 | 22 |
| 4 BA | 100 | ip | 1, 5 | 39 |
| | 75 | ip | 1, 5 | 41 |
| | 50 | ip | 1, 5 | 10 |
| 4 BM | 2.5[a] | ip | 1, 3 | 67 |
| | 1.25 | ip | 1, 2, 3 | 43 |

a = Maximum tolerated dose

b = Two independent experiments

2, 4 BA = 2, 4-dichloro-3, 5-dinitrobenzoic acid (C1)

4BA = 4-chloro-3, 5-dinitrobenzoic acid (C4)

4 BM = 4-chloro-3, 5-dinitrobenzoic acid methyl ester (C6)

TABLE 4

Anti-tumour activity against MAC13 murine colon adenocarcinoma 12 days after im transplant. Drugs dissolved in corn oil. Dosage is per day.

| Compound | Dose (mg/kg) | Route | Schedule (days) | % Tumour Weight inhibition |
|---|---|---|---|---|
| 2, 4 BA | 75[a] | ip | 1, 4, 5 | 45 |
| 2, 4 BA | 50 | ip | 1, 2, 3, 4, 5, 6, 7, 8, 9 | 39 |
| 2, 4 BA | graph[1] | ip | graph[1] | graph[1] |
| 2 BA | graph[2] | ip | graph[2] | graph[2] |

a = Maximum tolerated dose

2, 4 BA = 2, 4-dichloro-3, 5-dinitrobenzoic acid[1] (C1)

2 BA = 2-chloro-5-nitrobenzoic acid (C17)

(1: see Figure 2 of the drawings; 2: see figure 3 of the drawings)

TABLE 5

Anti-tumour activity against MAC16, murine colon adenocarcinoma sc transplanted on day 11 after the beginning of treatment with 2, 4-dichloro-3, 5-dinitrobenzoic acid (2, 4 BA; C1). Drug dissolved in corn oil. The tumour volumes were at least 40mm$^3$ at the beginning of the treatment. 6 animals per group. Dosage is per day.

| Compound | Dose (mg/kg) | Route | Schedule (days) | % Tumour Weight Inhibition |
|---|---|---|---|---|
| 2, 4 BA | 75[a] | ip | 1, 2, 5, 8 | 88 |
| | 50 | ip | 1, 2, 4, 5, 8 | 91 |

a = Maximum tolerated dose

TABLE 6

| Anti-tumour activity of C17 against B16 murine melanoma 12 days after sc transplant on female C57/black mice. 6 animals per group. Dosage is per day and is ip. | | | | |
|---|---|---|---|---|
| Code | Compound | Dose (mg/kg) | Schedule (days) | % Tumour Weight Inhibition |
| C17 | 2-chloro-5-nitrobenzoic acid | 700 | 1, 2, 3, 4, 5, 6 | 62 |

Anti-tumour activity and toxicity studies have additionally been completed for the following compounds with broadly satisfactory results:-

C22 2, 5-dichloro-4-nitrobenzoic acid
C23 2, 4-dichloro-5-nitrobenzoic acid
C24 2, 6-dichloro-4-nitrobenzoic acid
C26 2-hydroxy-5-nitrobenzoic acid
C27 3, 5-dichloro-4-nitrobenzoic acid

In addition, the following primary assay was used to investigate the anti-viral activity of compounds in accordance with the invention.

Primary Assay

(i) *Acute Infection Assay.* High titre virus stocks of the human immunodeficiency virus HIV-$1_{RF}$ were grown in H9 cells with RM1 1640 (Flow laboratories) supplemented with 10% fetal calf serum, penicillin (100IU/ml). Cell debris was removed by low speed centrifugation, and the supernatant stored at -70°C until required. In a typical assay C8166 T-lymphoblastoid CD4+ cells were incubated with 10xTCID50 HIV-$1_{RF}$ at 37°C for 90 minutes and then washed three times with phosphate buffered saline (PBS). Cell aliquots (2 x $10^5$) were resuspended in 1.5ml growth medium in 6ml tubes, and active compounds in log dilutions [200μM to 0.2μM] were added immediately. 20mM stock solutions of each compound were made up in 70% alcohol. The compounds were stored as a powder and made up freshly in distilled water before each experiment or were stored as a 90 mM stock solution in 70% alcohol. The final concentration of alcohol in the tissue culture medium was 1%. The cells were then incubated at 37°C in 5% $CO_2$. At 72 hours post-infection 200μl of supernatant was taken from each culture and assayed for HIV (Kingchington et al, 1989, Robert et al 1990) using an antigen capture ELISA which recognizes all the core proteins equally (Coulter Electronics, Luton, UK). The following controls were used: supernatants taken from uninfected and infected cells, infected cells treated with AZT (Roche Products UK Ltd) and ddC (Roche) and RO31-8959 (Roche) an inhibitor of HIV proteinase. The $IC_{50}$ activities of 8959, AZT and ddC in infected cells were 1, 10, 20 nM and 200nM respectively (accompanying Figure 2). The ELISA plates were read with a spectrophotometer. Compounds were tested in duplicate at each concentration, and the data shown is the average of at least two assays. This assay assesses the activity of compounds by measuring their inhibition of HIV core antigen levels.

(ii) *Chronically Infected Cell Assay.* Chronically infected cells (H9rf) were washed three times to remove extracellular virus and incubated with the active compounds (200-0.2 μM) for four days. HIV-1 antigen in the supernatant was then measured using an ELISA.

To test for compound toxicity, uninfected H9 cells were incubated with the compounds for four days. Supernatants were discarded and the cells resuspended in 200μl pg growth medium containing $^{14}C$ protein hydrolysate. After 6 hours the cells were harvested and the $^{14}C$ incorporation measured.

(iii) *Toxicity Assay.* To test for compound toxicity, aliquots of 2 x $10^5$ of uninfected cells were cultured with the compounds in the same dilutions for 72 hours. The cells were then washed with PBSA and resuspended in 200μl of growth medium containing $^{14}C$ protein hydrolysate. After 12, hours the cells were harvested and the $^{14}C$ incorporation measured. Uninfected, untreated cells were used as controls. Toxicity is expressed as inhibition of uptake of $^{14}C$ protein hydrolysate.

The results of these assays are summarized in Table 7 below. The $IC_{50}$ is the drug concentration that causes a 50% reduction in HIV core antigen levels as detected by the Coulter P24 antigen assay and is determined by doubling dilutions of supernatant taken from tubes containing untreated acutely infected cells. The $CD_{50}$ is the concentration of drug that causes a 50% inhibition of cells as measured by $^{14}C$ protein hydrolysate uptake. The therapeutic index (TI) is determined by dividing the $CD_{50}$ by the $IC_{50}$.

TABLE 7

| Code | Compound | $IC_{50}$ | $CD_{50}$ | TI |
|---|---|---|---|---|
| C4 | 4-chloro-3, 5-dinitrobenzoic acid | 30μm | 70μm | 2.33 |

Following the same methodology, more extensive assays were performed as reported in Tables 8 below.

TABLE 8.1

| STRUCTURE ACTIVITY RELATIONSHIP AGAINST HIV VIRUS | |
|---|---|
| CODE | COMPOUNDS |
| C1 | 2, 4-dichloro-3, 5-dinitrobenzoic acid |
| C3 | 2, 4-dichloro-3, 5-dinitrobenzoic acid methyl ester |
| C4 | 4-chloro-3, 5-dinitrobenzoic acid |
| C6 | 4-chloro-3, 5-dinitrobenzoic acid methyl ester |
| C7 | 2-chloro-3, 5-dinitrobenzoic acid |
| C8 | 2-chloro-3, 5-dinitrobenzoic acid methyl ester |
| C9 | 4-chloro-3-nitrobenzoic acid |
| C10 | 2-chloro-4-nitrobenzoic acid |
| C11 | 3, 4-dichlorobenzoic acid |
| C12 | 2, 5-dichlorobenzoic acid |
| C17 | 2-chloro-5-nitrobenzoic acid |

TABLE 8.2

| | IC50 (Antiviral) | CC50 (Toxicity) | SI (Selectivity Index) |
|---|---|---|---|
| Against HIV-I IIIB | | | |
| C1 | 5 | 70 | 14 |
| | 36 | 70 | 2 |
| | 33 | 70 | 2 |
| | 35 | 60 | 2 |
| **Average** | **27** | **70** | **3** |
| C1 | 10 | 30 | 3 |
| | 2.5 | 20 | 8 |

| | | | |
|---|---|---|---|
| Against HIV-IRF | | | |
| C1 | 7 | 60 | 8.5 |
| | - | - | 56 |
| | 16 | 56 | 3.5 |
| **Average** | **11.5** | **57** | **5** |
| Against Chronically infected cells | | | |
| C1 | 16 | 30 | 2 |
| | 16 | 95 | 6 |
| **Average** | **16** | **63** | **4** |
| Against HIV-1-IIIB | | | |
| C2 | 2 | 70 | 35 |
| C3 | 0.3 | 7 | 23 |
| C4 | 40 | 100 | 2.5 |
| | 30 | 70 | 2.3 |
| **Average** | **35** | **85** | **2.4** |
| C5 | 5 | 50 | 10 |
| C6 | 5 | 60 | 12 |
| C7 | 23 | 150 | 6 |
| | 5 | >200 | >10 |
| **Average** | **22** | **>175** | **8** |
| C7 | >1 | 35 | >35 |
| | 10 | 30 | 3 |
| C8 | 10 | 60 | 5 |
| C9 | >200 | >200 | - |
| C10 | >200 | >200 | - |
| C11 | >200 | >200 | - |
| C12 | >200 | >200 | - |
| Against HIV-I IIIB | | | |
| C17 | >1 | >1000 | >1000 |
| | 5 | >1000 | >200 |

Patient Tests

A pilot study was conducted for safety, pharmacokinetics and preliminary activity of C17 in patients with HIV-1 infection and HIV-related Kaposi's sarcoma. In the pilot study, 10 patients were enrolled but only 6 were eligible for evaluation. These patients were positive for serum antibody to HIV-1 as determined by both enzyme-linked immunosorbent assay (ELISA) and Western Blot. These patients had WHO Clinical Stage 2 to 3 for HIV infection and disease and fulfilled the inclusion and exclusion criteria as per the Clinical Trial Protocol. Their CD4 ranged from 0.072 to 0.812 x $10^3$ cells/$mm^3$ (normal range 1.0 x 1.3 x $10^3$ cells/$mm^3$). Patient 6 had AIDS-related Kaposi's sarcoma confirmed on histology.

C17 was prepared for intravenous administration under GMP (good manufacturing practice) in strength of 40 mg/ml following the method set forth below for producing a 250mg/ml solution, additional water being added at Step 7:-

Method of Preparation

1. To 275ml water for injection add 266.4ml 4N sodium hydroxide solution and mix.
2. Add with stirring 182.5g of active compound.
3. Add additional 4N sodium hydroxide if needed to obtain solution.
4. Filter solution through a Sterivex GV 0.22μm filter.
5. Dilute to approximately 680ml with water for injection.
6. Adjust to pH 7.2-7.6 with hydrochloric acid.
7. Make-up to 730ml water for injection. Adjust pH if necessary.
8. Transfer solution to aseptic room and filter through Sterivex GV 0.22μm filter into injection vials. Seal with rubber stoppers and aluminium closures.
9. Effect terminal sterilization by autoclave.

The patients received 10mg/kg body weight/day of C17 by deep intramuscular injection daily for 5 days weekly. The CD4 counts were estimated before and at about 2 weeks after the beginning of the therapy.

The CD4 counts in all 6 patients showed an increase which was associated with clinical improvements in patient general condition including weight gain and a marked decrease in opportunistic infections and diaorrhea. Patient observations are shown in Tables 9 below.

In the case of Patient 6, the sarcoma lesions disappeared.

Murine Haematology

Preliminary data on the administration of C17 in mice and rats showed increases in red blood cell count, haemoglobin concentration, macrophage count, white blood cell count (WBC), lymphocyte count and haematocrit (HTC) on day 7 when maximum tolerated dose (MTD) was given by 5 consecutive daily intravenous injections. These values returned to normal on day 14. The MTD values were 950mg and 900mg/kg body weight/day respectively in mouse and rat. The data are reported in Figures 4 to 11.

TABLE 9.0 (Summarizing Tables 9.1 to 9.6)

PATIENTS 1 TO 6 IMMUNOLOGY:-

| | PATIENT 1 | | 2 | | 3 | | 4 | | 5 | | 6 | |
|---|---|---|---|---|---|---|---|---|---|---|---|---|
| | Before | After | Before | After | Before | After | Before | After | Before | After | Before | After |
| Lymphocytes x $10^3 mm^{-3}$ | 2.1 | 2.0 | 2.0 | 1.9 | 0.9 | 1.1 | 1.8 | 2.0 | 2.8 | 3.1 | 2.1 | 1.9 |
| CD4 % | 19 | 38 | 18 | 27 | 8 | 17 | 6 | 22 | 29 | 33 | 18 | 23 |
| CD4 ABS/$mm^3$ | 0.399 | 0.760 | 0.360 | 0.513 | 0.072 | 0.187 | 0.108 | 0.440 | 0.812 | 1.023 | 0.378 | 0.437 |
| CD8 ABS/$mm^3$ | 0.567 | 0.840 | 0.940 | 0.874 | 0.270 | 0.682 | 1.350 | 1.260 | 1.456 | 1.550 | 1.218 | 0.931 |
| CD4/CD8 | 0.70 | 0.90 | 0.38 | 0.59 | 0.27 | 0.27 | 0.08 | 0.35 | 0.56 | 0.66 | 0.31 | 0.47 |

TABLE 9.1

PATIENT 1 IMMUNOLOGY:-

| DAY | 1 | 20 |
|---|---|---|
| Lymphocytes x $10^3 mm^{-3}$ | 2.1 | 2.0 |
| CD4 % | 19 | 38 |
| CD4 ABS/$mm^3$ | 0.399 | 0.760 |
| CD8 ABS/$mm^3$ | 0.567 | 0.840 |
| CD4/CD8 | 0.70 | 0.90 |

TABLE 9.2

PATIENT 2 IMMUNOLOGY:-

| DAY | 1 | 14 |
|---|---|---|
| Lymphocytes x $10^3 mm^{-3}$ | 2.0 | 1.9 |
| CD4 % | 18 | 27 |
| CD4 ABS/$mm^3$ | 0.360 | 0.513 |
| CD8 ABS/$mm^3$ | 0.940 | 0.874 |
| CD4/CD8 | 0.38 | 0.59 |

TABLE 9.3

PATIENT 3 IMMUNOLOGY:-

| DAY | 1 | 20 |
|---|---|---|
| Lymphocytes x $10^3 mm^{-3}$ | 0.9 | 1.1 |
| CD4 % | 8 | 17 |
| CD4 ABS/$mm^3$ | 0.072 | 0.187 |
| CD8 ABS/$mm^3$ | 0.270 | 0.682 |
| CD4/CD8 | 0.27 | 0.27 |

TABLE 9.4

PATIENT 4 IMMUNOLOGY:-

| DAY | 1 | 14 |
|---|---|---|
| Lymphocytes x $10^3 mm^{-3}$ | 1.8 | 2.0 |
| CD4 % | 6 | 22 |
| CD4 ABS/$mm^3$ | 0.108 | 0.440 |
| CD8 ABS/$mm^3$ | 1.350 | 1.260 |
| CD4/CD8 | 0.08 | 0.35 |

TABLE 9.5

PATIENT 5 IMMUNOLOGY:-

| DAY | 1 | 21 |
|---|---|---|
| Lymphocytes x $10^3mm^{-3}$ | 2.8 | 3.1 |
| CD4 % | 29 | 33 |
| CD4 ABS/$mm^3$ | 0.812 | 1.023 |
| CD8 ABS/$mm^3$ | 1.456 | 1.550 |
| CD4/CD8 | 0.56 | 0.66 |

TABLE 9.6

PATIENT 6 IMMUNOLOGY:-

| DAY | 1 | 34 |
|---|---|---|
| Lymphocytes x $10^3mm^{-3}$ | 2.1 | 1.9 |
| CD4 % | 18 | 23 |
| CD4 ABS/$mm^3$ | 0.378 | 0.437 |
| CD8 ABS/$mm^3$ | 1.218 | 0.931 |
| CD4/CD8 | 0.31 | 0.47 |

## Claims

1. A compound of the following general formula for use in the treatment of prophylaxis or therapy of neoplasm or viral infection:-

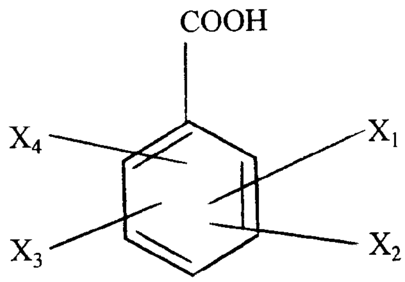

I

wherein $X_1$ and $X_2$ are, each independently, hydrogen or chloro and wherein $X_3$ and $X_4$ are, each independently, hydrogen or nitro, provided that at least two of $X_1$, $X_2$, $X_3$ and $X_4$ are other than hydrogen, or a salt or ester thereof.

2. A compound as claimed in Claim 1 wherein $X_1$ is chloro, $X_2$ is chloro or hydrogen, $X_3$ is nitro and $X_4$ is nitro or hydrogen.

3. A compound as claimed in Claim 2 wherein $X_1$ and $X_2$ are other than 4-chloro.

4. A compound as claimed in Claim 1 or Claim 2 wherein $X_1$ and $X_2$ together represent 2-chloro-, 4-chloro-, 2,5-dichloro-, 2, 4-dichloro-, 2, 6-dichloro-, 3, 5-dichloro- or 3, 4-dichloro- and wherein $X_3$ and $X_4$ together represent two hydrogen atoms, 3-nitro-, 4-nitro-, 5-nitro-, 2, 4-dinitro- or 3, 5-dinitro-, provided that:-
   (i) when $X_3$ and $X_4$ together represent 3, 5-dinitro-, $X_1$ and $X_2$ together represent 2-chloro-,
   (ii) when $X_3$ and $X_4$ together represent 5-nitro-, $X_1$ and $X_2$ together represent 2-chloro- or 2, 4-dichloro-,
   (iii) when $X_3$ and $X_4$ together represent two hydrogen atoms, X1 and $X_2$ together represent 3, 4-dichloro-,
   (iv) when $X_3$ and $X_4$ together represent 4-nitro-, $X_1$ and $X_2$ together represent 2, 5-dichloro-, 3, 5-dichloro-2-chloro- or 2, 6-dichloro-,
   (v) when $X_3$ and $X_4$ together represent 3-nitro-, $X_1$ and $X_2$ together represent 4-chloro-.

5. A compound as claimed in Claim 4 wherein $X_1$, $X_2$, $X_3$ and $X_4$ are as there defined subject to provisos (I), (ii), (iv) and (v) and subject to the further proviso that when $X_3$ and $X_4$ together represent 5-nitro-, $X_1$ and $X_2$ together represent 2, 4-dichloro-.

6. 2-chloro-5-nitro-benzoic acid.

7. A compound as claimed in Claim 1 and as set forth by name below or an ester or salt thereof:-
   7.1 2, 4-dichloro-3, 5-dinitrobenzoic acid
   7.2 4-chloro-3, 5-dinitrobenzoic acid
   7.3 2-chloro-3, 5-dinitrobenzoic acid
   7.4 2, 5-dichlorobenzoic acid
   7.5 2, 4-dinitrobenzoic acid
   7.6 3, 5-dinitrobenzoic acid
   7.7 2, 5-dichloro-4-nitrobenzoic acid
   7.8 2, 4-dichloro-5-nitrobenzoic acid
   7.9 2, 6-dichioro-4-nitrobenzoic acid
   7.10 3, 5-dichloro-4-nitrobenzoic acid
   7.10 4-chloro-3-nitrobenzoic acid
   7.12 2-chloro-4-nitrobenzoic acid
   7.13 3, 4-dichlorobenzoic acid

8. A pharmaceutical composition for use in the treatment by prophylaxis or therapy of neoplasm or viral infection, the composition comprising a compound as claimed in any preceding claim together with a pharmaceutically-acceptable carrier or diluent.

9. A composition as claimed in Claim 8 wherein the carrier or diluent is aqueous.

10. A composition as claimed in Claim 8 wherein the carrier is a solid excipient.

11. A composition as claimed in Claim 8 which is in tablet, capsule, powder, solution or suspension form.

12. A composition as claimed in any one of Claims 8 to 11 which is in unit dosage form.

13. A composition as claimed in any one of Claims 8 to 12 and comprising 2-chloro-5-nitrobenzoic acid, or a salt or ester thereof, encapsulated by a soft gel capsule or contained by a sustained release drug delivery system.

14. A method of preparing a pharmaceutical composition as claimed in any one of Claims 8 to 13 which method comprises combining a compound as claimed in any one of Claims 1 to 7 with aqueous sodium hydroxide solution to form a solution, adjusting the pH of the solution to an alkaline pH of not more than 8 by addition to the solution of an acidic material, optionally diluting the pH-adjusted solution with physiologically injectable water and sterilizing the solution.

15. A method of treatment by prophylaxis or therapy of neoplasm or viral injection in mammalian subjects, which method comprises administering to a mammalian subject an effective dose or dosage regime of a compound as claimed in any one of Claims 1 to 7.

16. Use of a compound as claimed in any one of Claims 1 to 7 for the preparation of a medicament for the treatment by prophylaxis or therapy of neoplasm or viral infection in mammalian subjects.

17. Use of a compound as claimed in any one of Claims 1 to 7 for the treatment of Kaposi's sarcoma.

18. Use of a compound as claimed in any one of Claims 1 to 7 for the preparation of a medicament for the treatment of Kaposi's sarcoma.

19. A method of treatment of mammals for the prophylaxis of Kaposi's sarcoma which method comprises administering to the patient an effective dose of a compound as claimed in any one of Claims 1 to 7.

20. A pharmaceutical composition in the form of enteric coated tablets comprising a compound as claimed in any one of Claims 1 to 7 in strength 100 to 400mg, the compound being formulated as a mixture comprising the compound together with an inert pharmaceutically-acceptable solid tabletting carrier.

21. A pharmaceutical composition in the form of an injectable solution or dispersion of a compound as claimed in any one of Claims 1 to 7 in an aqueous or other injectable liquid carrier in a concentration of less than 250mg/ml.

FIG.1

AZT AND ddCyd
INTERNAL
CONTROLS

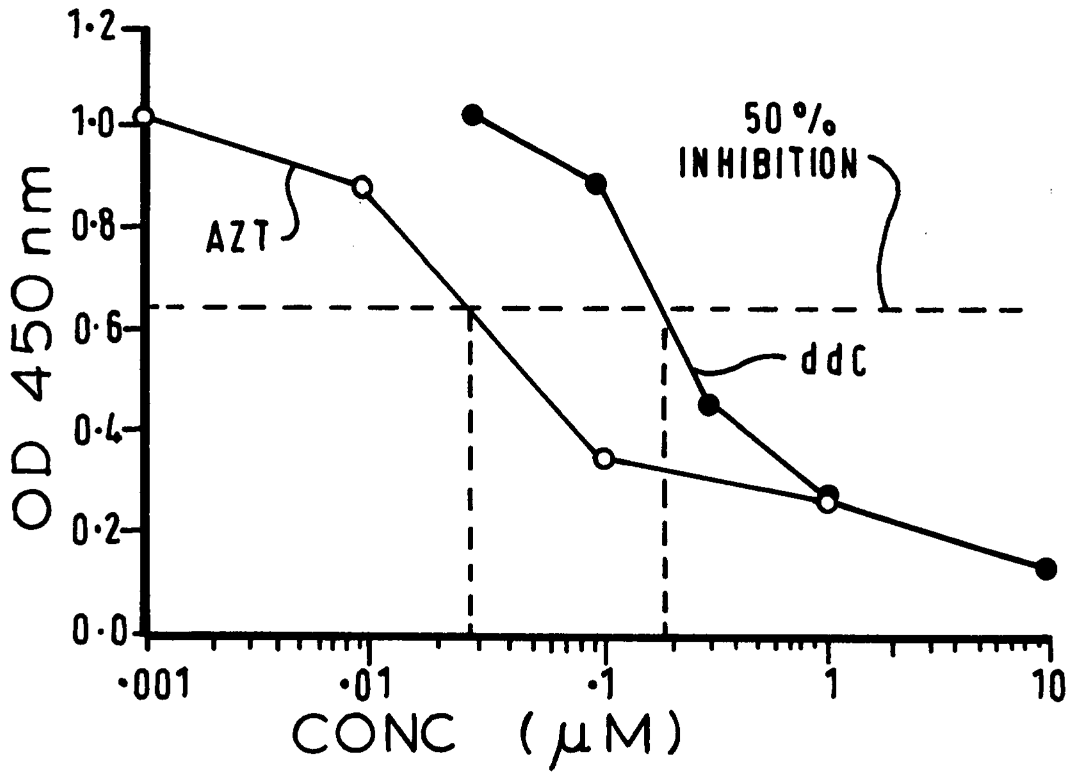

FIG. 2

Effect against MAC13 Murine Colon Adenocarcinoma Transplanted S C 6 animals per group

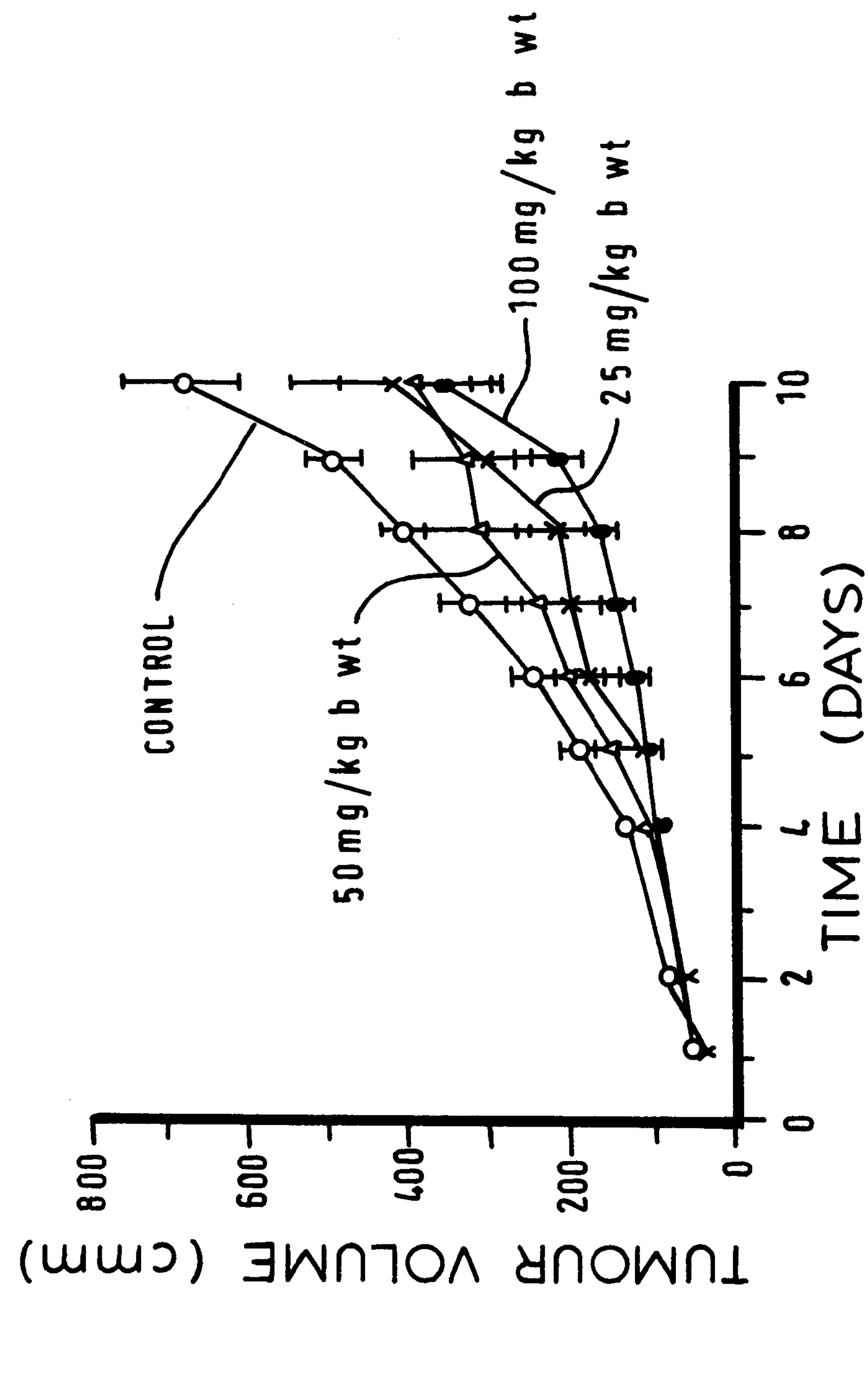

# FIG. 3

Effect against MAC 13 Murine Colon Adenocarcinoma Transplanted SC 6 animals per group

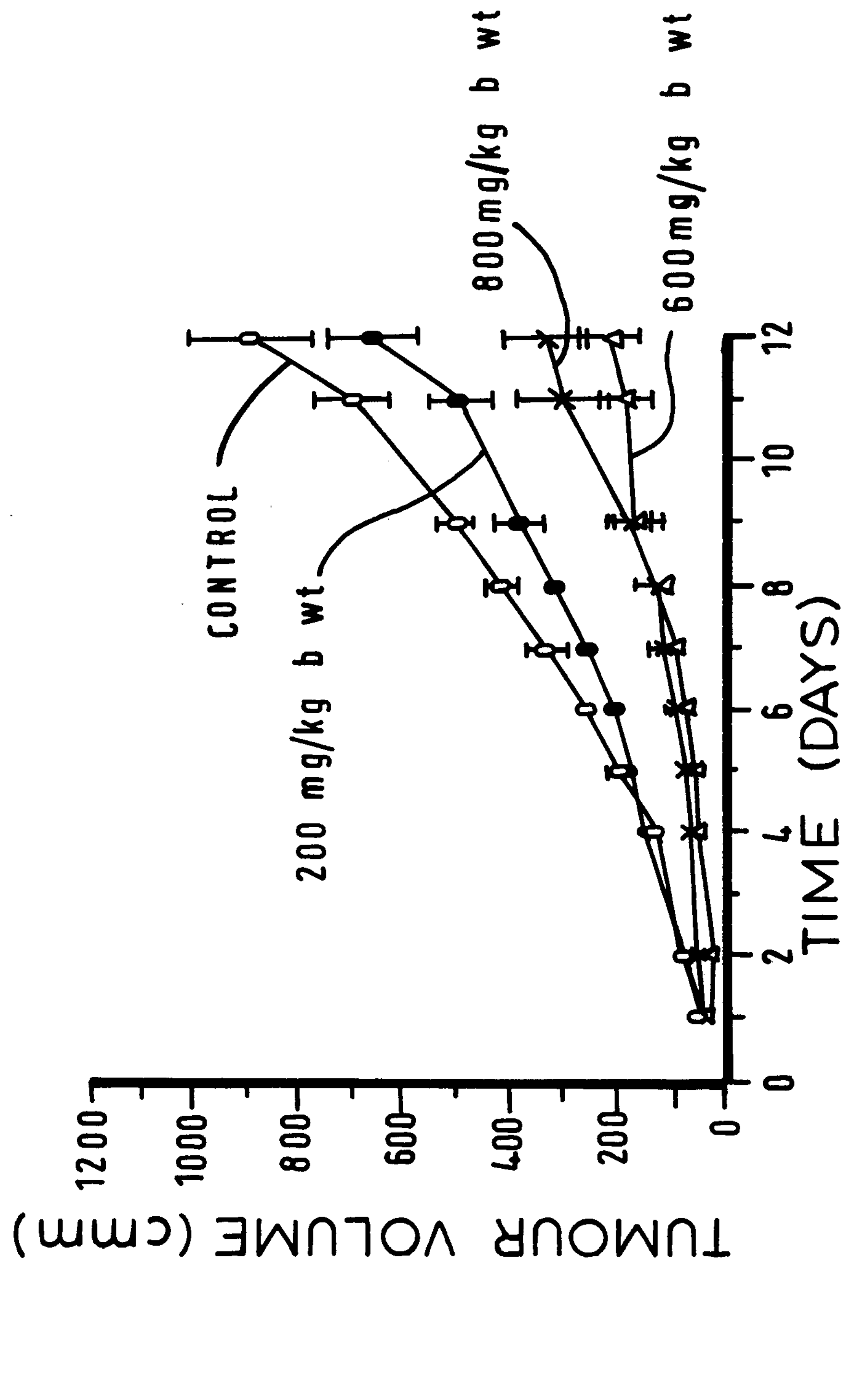

FIG. 4
Effect of C17 on rat Hemaglobin
after 3 and 7 days

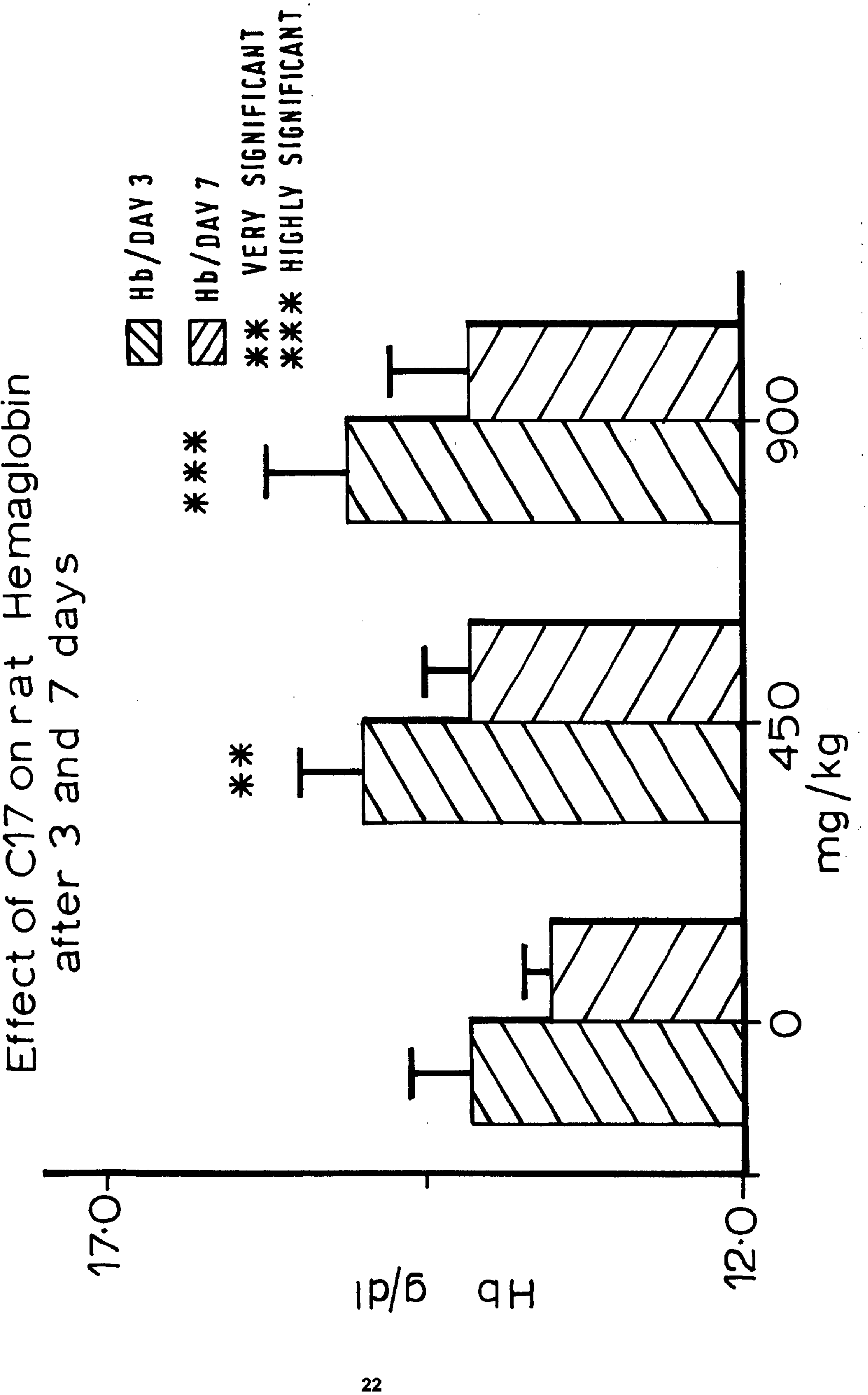

**FIG.5**

Effect of C17 on rat RBCs after 3 and 7 days

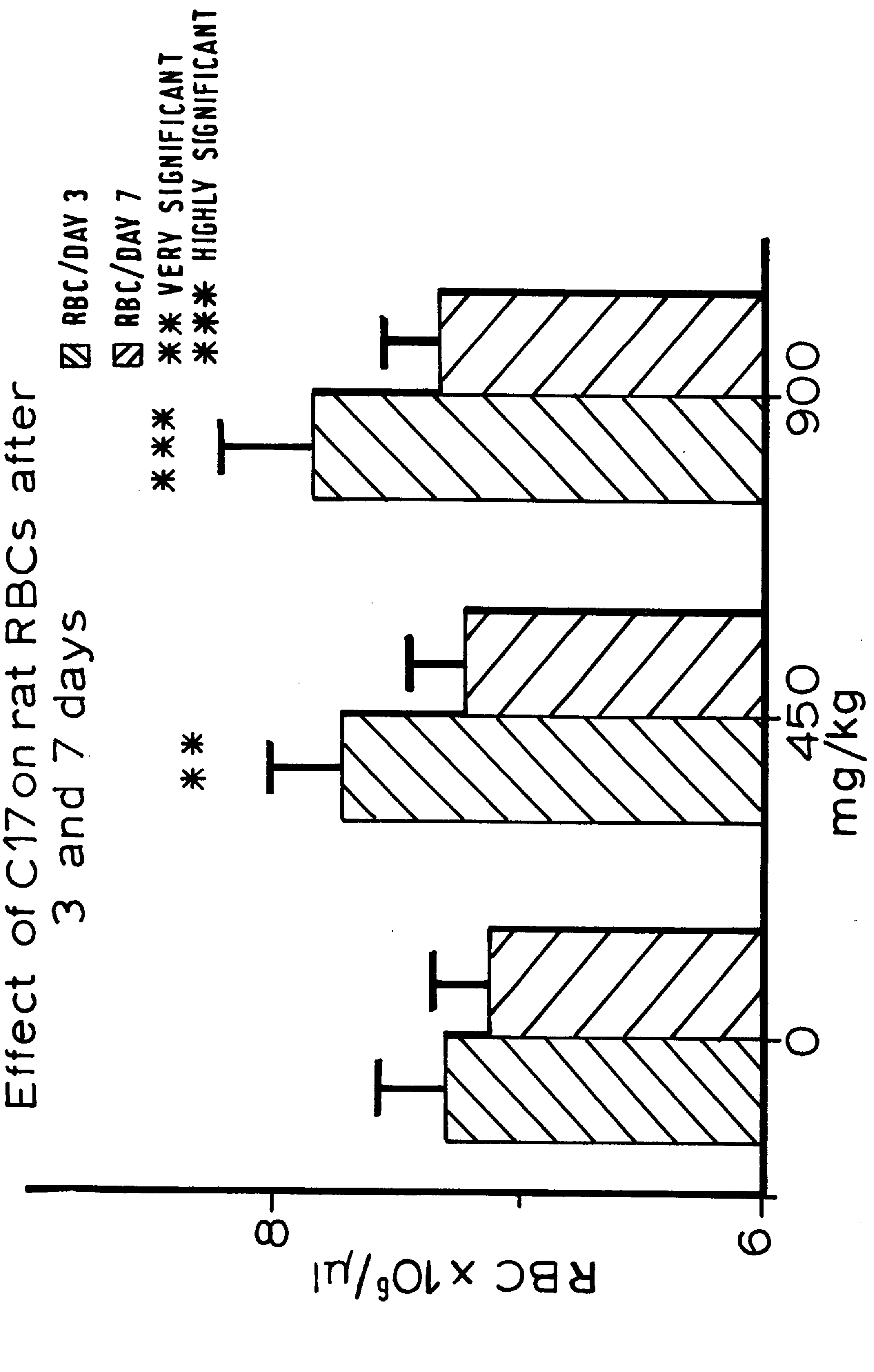

FIG.6

Effect of C17 on %HCT in rats after 3 and 7 days

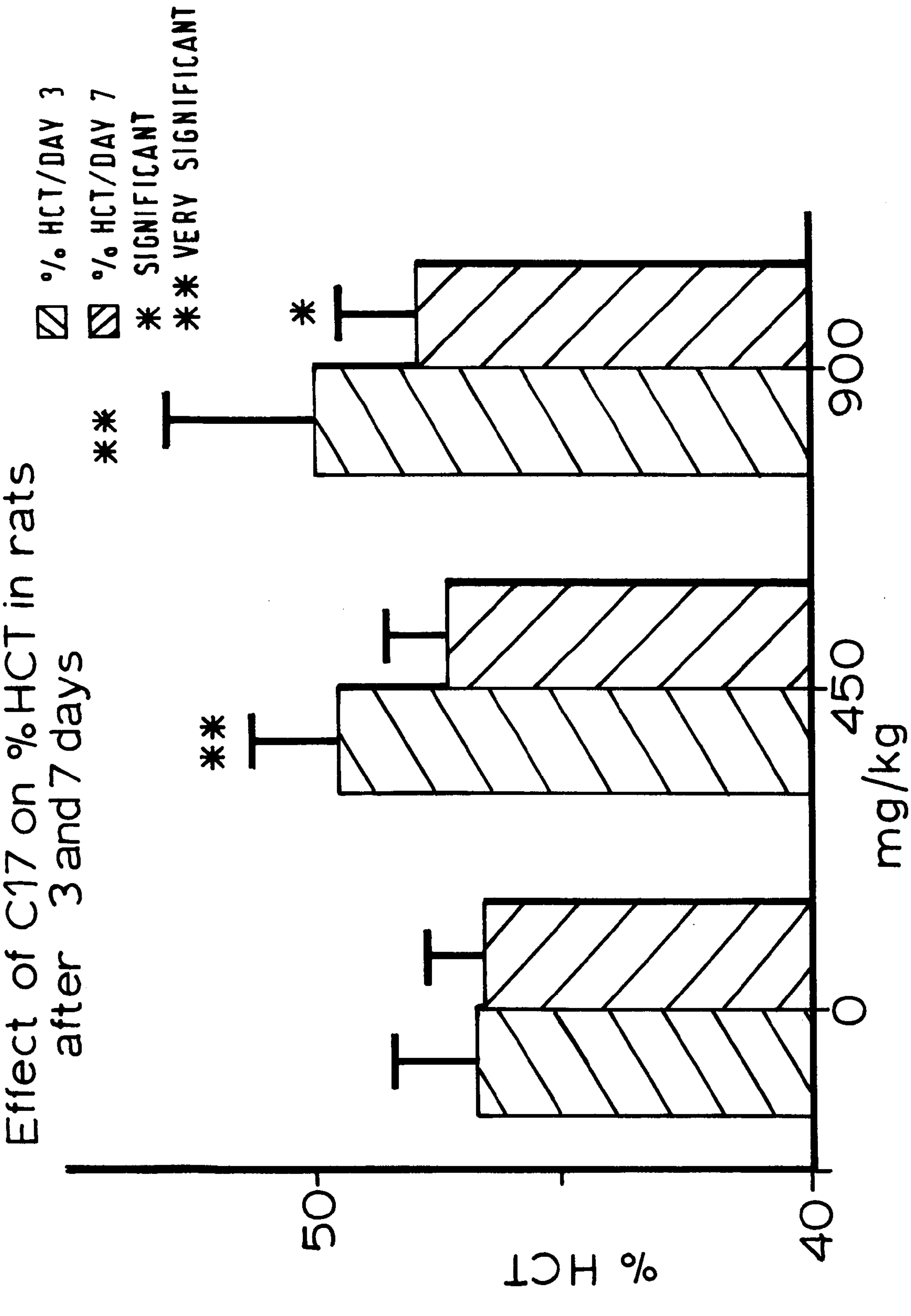

FIG.7

Effect of C17 on mouse macrophages after 7 and 14 days

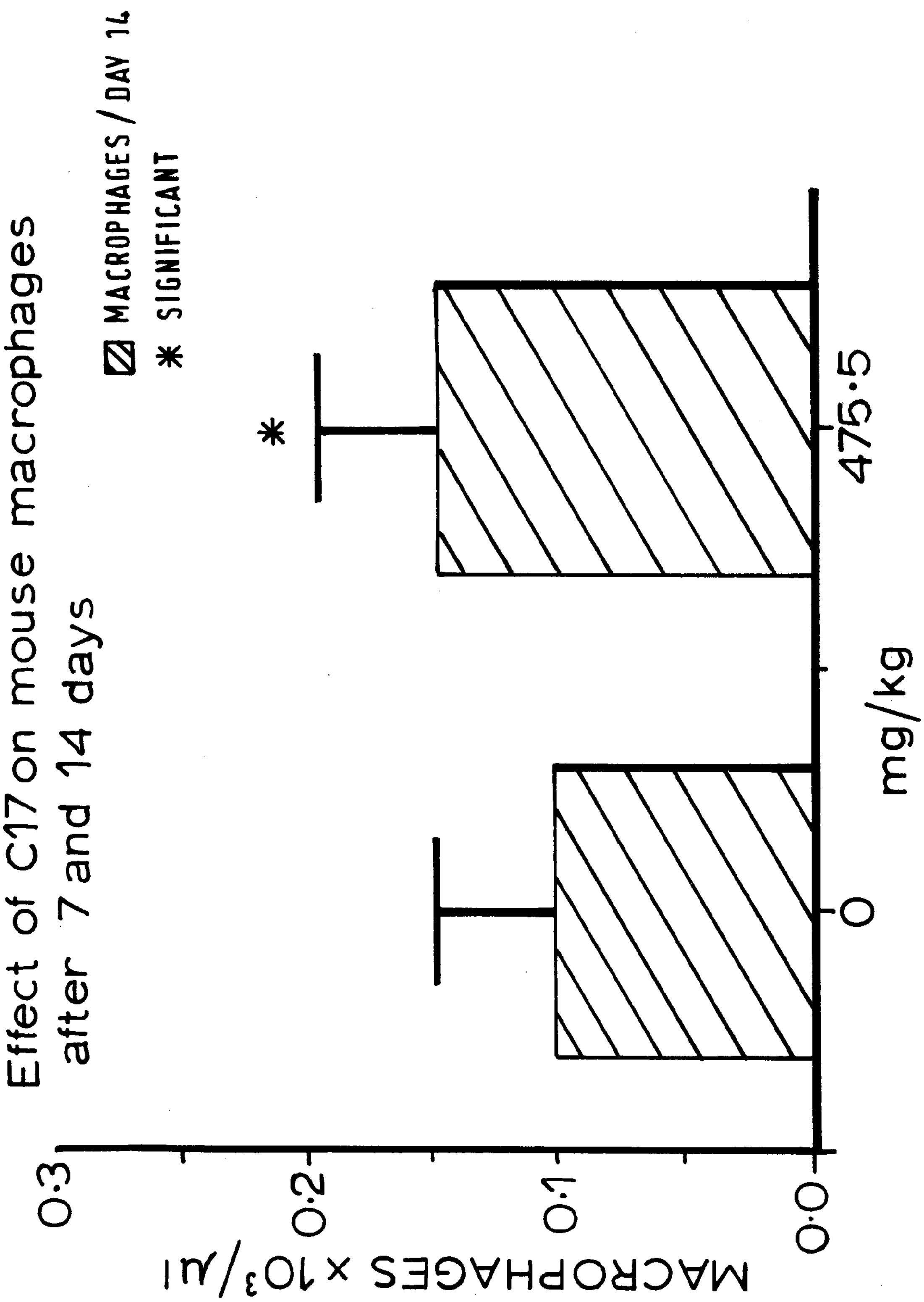

FIG. 8

Effect of C17 on mouse WBCs and lymphocytes after 7 and 14 days

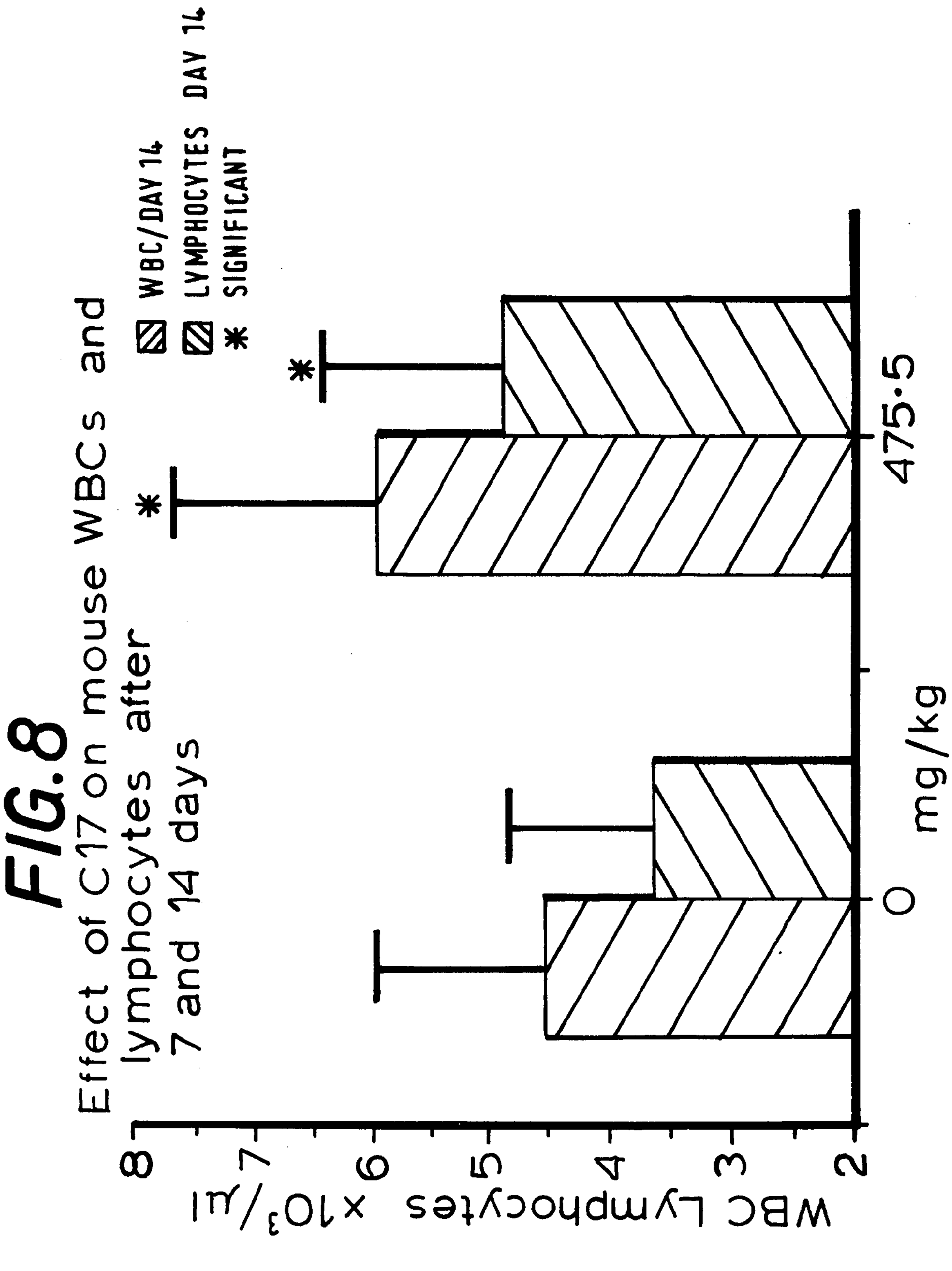

FIG.9

Effect of C17 on mouse HCT after 7 and 14 days

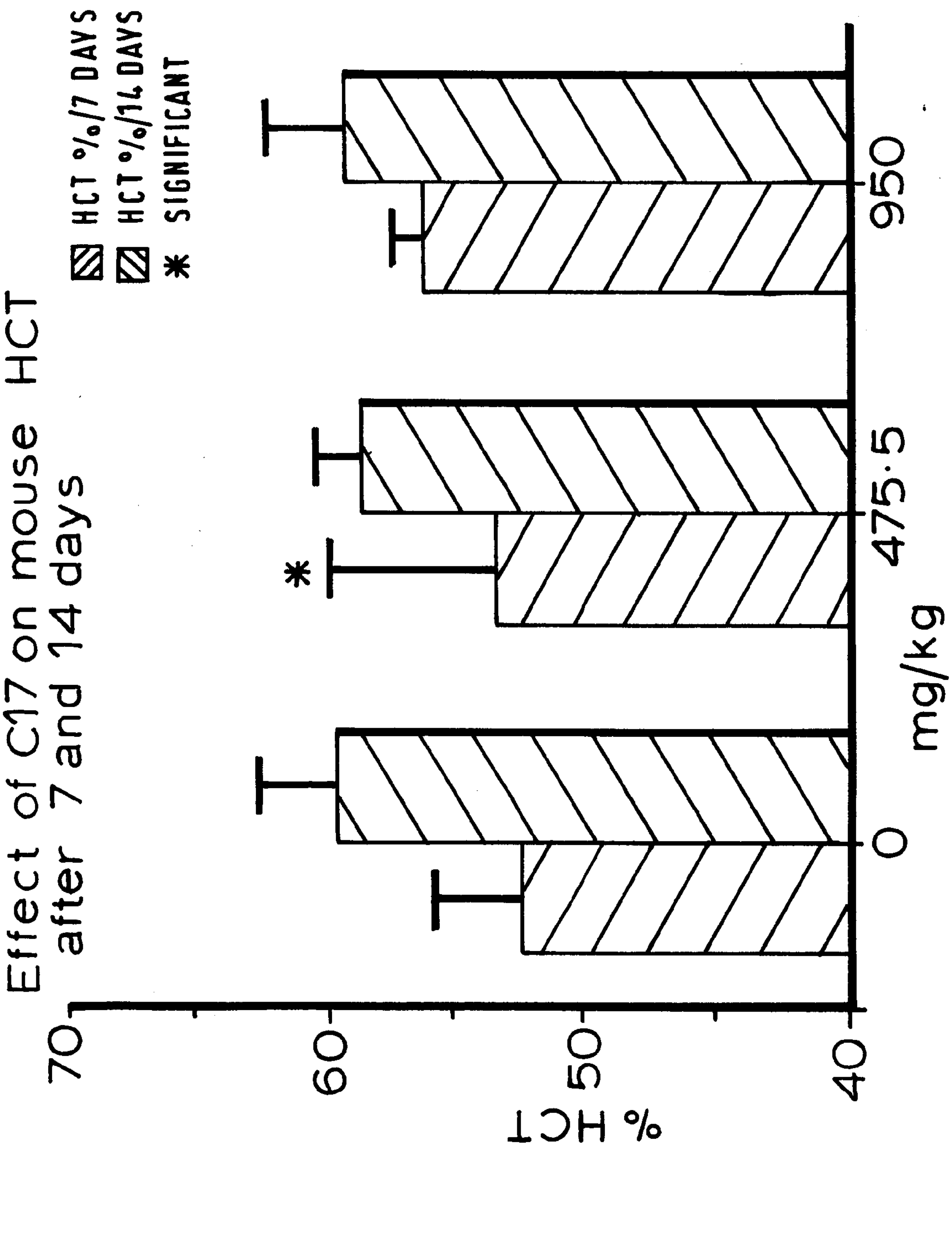

FIG. 10

Effect of C17 on mouse Hemaglobin levels at 7days and 14 days

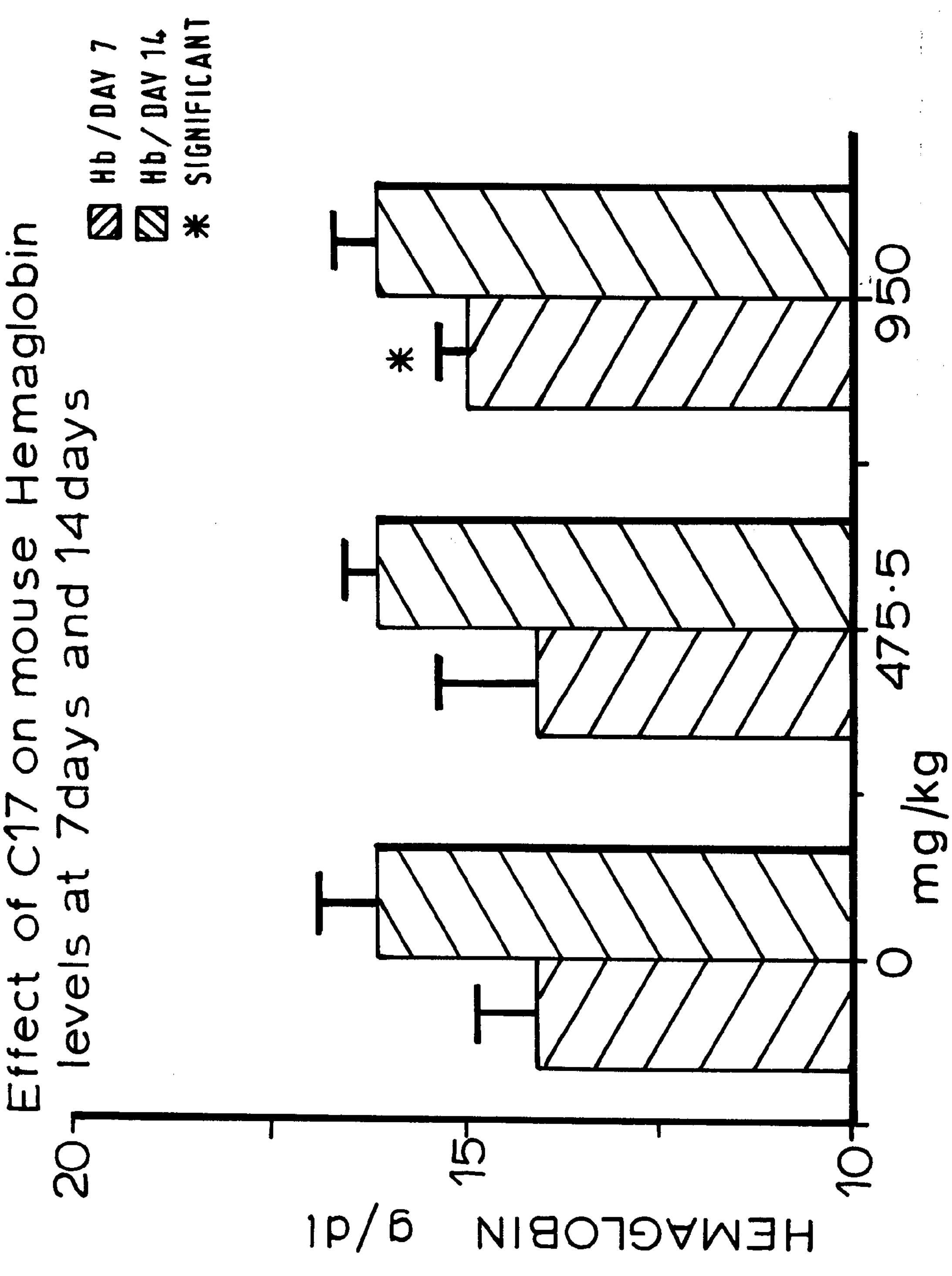

FIG. 11

Effect of C17 on mouse RBCs after 7 and 14 days

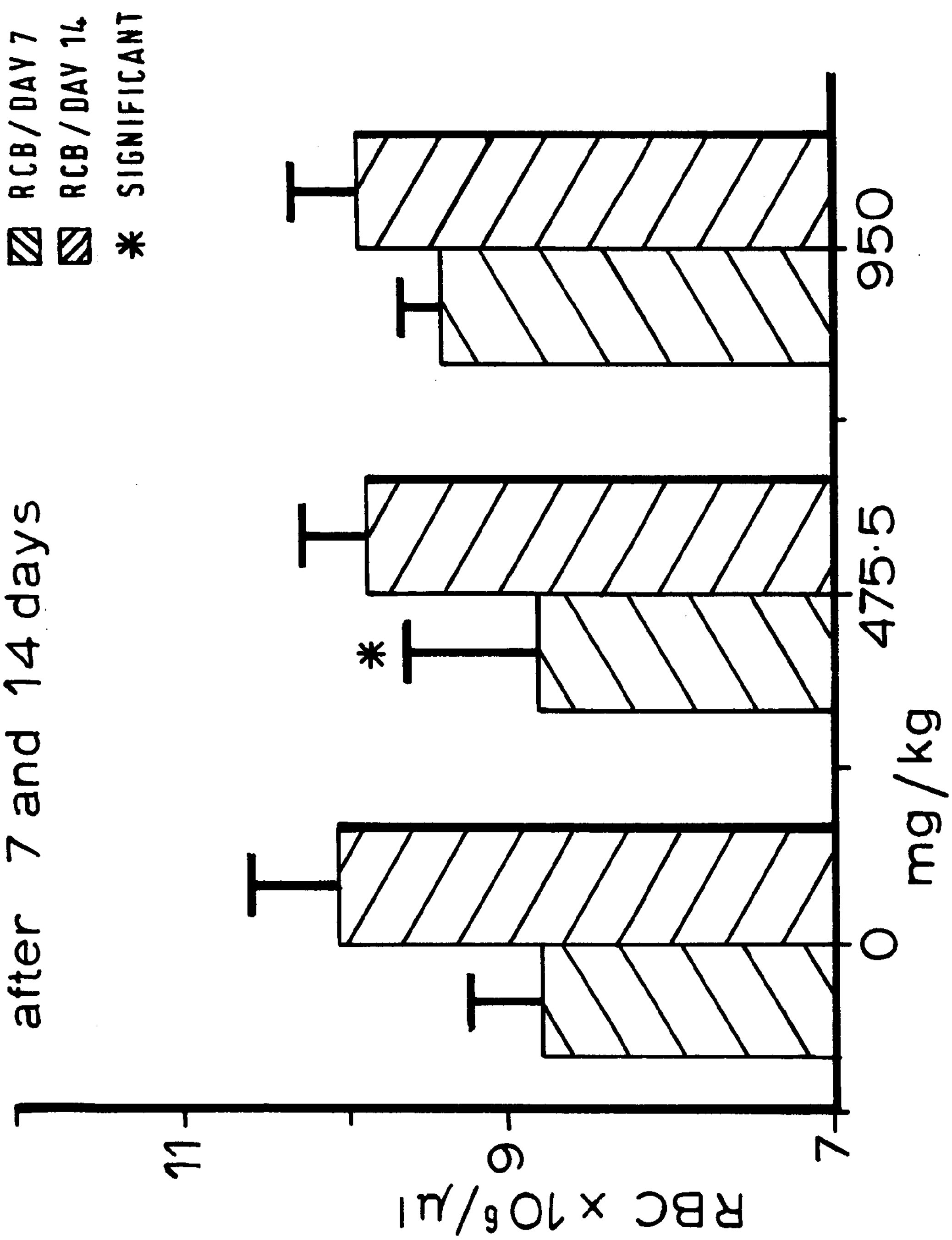

European Patent Office

# PARTIAL EUROPEAN SEARCH REPORT

which under Rule 45 of the European Patent Convention shall be considered, for the purposes of subsequent proceedings, as the European search report

Application Number EP 95 30 1797

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,P | WO-A-94 27584 (RADOPATH LIMITED)<br>* the whole document *<br>--- | 1-21 | A61K31/19 |
| X | DATABASE WPI<br>Derwent Publications Ltd., London, GB;<br>AN 86-180544<br>& JP-A-61 115 020 (NIPPON SHOKUBAI KAGAKU)<br>2 June 1986<br>* abstract *<br>--- | 1-21 | |
| X | GB-A-1 283 331 (SMITH KLINE & FRENCH LABORATORIES LIMITED)<br>* the whole document *<br>--- | 1-21 | |
| X | 'CATALOGUE HANDBOOK OF FINE CHEMICALS'<br>1992 , ALDRICH CHEMIE<br>* page 293 *<br>* page 311 *<br>* page 425 *<br>* page 429 *<br>* page 544 *<br>* page F16 - page F17 *<br>* page F19 *<br>---<br>-/-- | 1-21 | TECHNICAL FIELDS SEARCHED (Int.Cl.6)<br>A61K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 17 July 1995 | Theuns, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 95 30 1797 -C-

INCOMPLETE SEARCH

**REMARK :** Although claims 15 and 19 are directed to a method of treatment of the human/animal body (Article 52(4) EPC) the search has been carried out and based on the alleged effects of the compound/composition.

European Patent Office

# PARTIAL EUROPEAN SEARCH REPORT

Application Number
EP 95 30 1797

| DOCUMENTS CONSIDERED TO BE RELEVANT | | | CLASSIFICATION OF THE APPLICATION (Int.Cl.6) |
|---|---|---|---|
| Category | Citation of document with indication, where appropriate, of relevant passages | Relevant to claim | |
| X | DATABASE WPI<br>Derwent Publications Ltd., London, GB;<br>AN 85-320781<br>& JP-A-60 224 619 (ADEKA-ARGUS CHEM KK) 9 November 1985<br>* abstract *<br>--- | 1-21 | |
| X | MOL.TOXICOL.,<br>vol.1, no.1, 1987<br>pages 61 - 81<br>G.KLOPMAN ET AL. 'A COMPUTER AUTOMATED STUDY OF THE STRUCTURE-MUTAGENICITY RELATIONSHIPS OF NON-FUSED-RING NITROARENES AND RELATED COMPOUNDS'<br>* page 69 *<br>--- | 1-21 | TECHNICAL FIELDS SEARCHED (Int.Cl.6) |
| X | WO-A-91 15200 (W.O.AYUKO)<br>* the whole document *<br>--- | 1-21 | |
| X | WO-A-92 14454 (RADOPATH LIMITED)<br>* the whole document *<br>----- | 1-21 | |

EPO FORM 1503 03.82 (P04C10)